# EUROPÄISCHE PATENTANMELDUNG

(11) **EP 2 011 457 A1**
(43) Veröffentlichungstag der Anmeldung: **07.01.2009**
(21) Anmeldenummer: 08159690.0
(22) Anmeldetag: 04.07.2008
(51) Int. Cl.: A61F 5/02

(54) **Vorrichtung zur Wirbelsäulenentlastung**

(30) Priorität: 06.07.2007 AT 10482007
(71) Anmelder: Hodina, Günther, 5431 Kuchl (AT)
(72) Erfinder: Hodina, Günther, 5431 Kuchl (AT)
(74) Vertreter: Babeluk, Michael

(57) **Zusammenfassung**

Die vorliegende Erfindung betrifft eine Vorrichtung (3) zur Wirbelsäulenentlastung einer auf einem Sitz (1), insbesondere einem Fahrzeugsitz, sitzenden Person (2), wobei die Vorrichtung (3) eine linke und eine rechte mit dem Sitz (1) verbundene höhenveränderliche Achselstütze (5', 5") aufweist, wobei jede Achselstütze (5', 5") auf einem vorzugsweise stangenartigen Halter (4', 4") angeordnet ist, wobei vorzugsweise der Halter (4', 4") am Sitzteil (1a) des Sitzes (1) befestigt ist. Um uneingeschränkte Bewegungsfreiheit der Person (2) zu ermöglichen, ist vorgesehen, dass die Stützenhöhen (h', h") der Achselstützen (5', 5"), vorzugsweise die Längen der Halter (4', 4"), bei einer Wankbewegung des Oberkörpers der Person (2) um eine Längsachse (10) des Sitzes (1) veränderbar sind, wobei die beiden Halter (4', 4") miteinander gekoppelt sind.

## Beschreibung

Die Erfindung betrifft eine Vorrichtung zur Wirbelsäulenentlastung einer auf einem Sitz, insbesondere einem Fahrzeugsitz, sitzenden Person, wobei die Vorrichtung eine linke und eine rechte mit dem Sitz verbundene höhenveränderliche Achselstütze aufweist, wobei jede Achselstütze auf einem vorzugsweise stangenartigen Halter angeordnet ist, wobei vorzugsweise der Halter am Sitzteil des Sitzes befestigt ist.

Es ist bekannt, dass beim Steuern von motorgetriebenen Fahrzeugen nicht nur die erwünschten Vor- und Rückwärtsbewegungen erreicht werden, sondern zum Beispiel durch Unebenheiten auf dem Gelände oder der Straße lästige und auch gesundheitsgefährdende Vertikalbewegungen resultieren, die auf den Fahrer übertragen werden. Weiterhin ist es bekannt, Gesundheitsschäden dadurch zu vermeiden, dass immer besser federnde und gedämpfte Fahrzeugsitze und Fahrerhäuser eingebaut werden und dass auch durch gefederte Fahrzeugachsen für möglichst viel gefederte Masse gesorgt wird. Trotzdem kommt es häufig zu Rücken- und Hüftgelenkschmerzen bei fahrenden Personen, insbesondere bei Berufskraftfahrern. Dies ist darauf zurückzuführen, dass die Trägheit des Oberkörpers der Person ein ständiges Zusammenstauchen der Wirbelsäule bei einer vertikalen Stoßbewegung verursacht, wenn Unebenheiten überfahren werden. Die Vertikalbewegung wird auf den Fahrersitz und auf den Fahrer übertragen. Die Federung und die Dämpfung der Masse im Gesäßbereich und Oberschenkel übernimmt der Sitz. Die Masse des Oberkörpers wird zunächst über die Wirbelsäule, besonders die Lendenwirbel, und dann über die Hüftgelenksknochen auf den Sitz abgefedert. Dies ruft die dauernde Belastung durch Zusammenstauchen dieser Körperteile hervor.

Aus der DE 199 10 373 A1 ist eine Vorrichtung zur Entlastung der Wirbelsäule eines Fahrers bekannt, welche im Wesentlichen aus einem Korsett besteht, über welches der Oberkörper des Fahrers nach oben gehängt und somit entlastet wird. Knapp unterhalb des Kabinendaches des Fahrzeuges ist ein Gestell eingebaut, dass die vertikalen Bewegungen, die auf den Oberkörper übertragen werden, abfedert und dämpft. Eine Schnellverschlusseinrichtung ermöglicht dabei eine Verbindung zwischen Korsett und Federungsgestell. Eine ähnliche Vorrichtung, bei der der Oberkörper einer Person nach oben gezogen wird, ist aus der WO 89/06149 A1 bekannt.

Auch in der DE 197 28 755 A1 wird eine Halte- bzw. Hängevorrichtung für Fahrzeuge vorgeschlagen, welche darauf abzielt, die Wirbelsäule zu entlasten.

Aus der DE 197 02 171 A1 ist ein Sitz mit Wirbelsäulenentlastung bekannt, welcher Achselstützen aufweist, mit deren Hilfe sich der Benutzer vom eigentlichen Sitz leicht abheben und so Rücken und Wirbelsäule strecken und somit entlasten kann. Die Achselstützen sind über einen Gelenkzapfen an der Rückenlehne des Sitzes schwenkbar gelagert und können in der Höhe verstellt werden. Nachteilig ist, dass bei einem Unfall, beispielsweise einem mehrfachen Auffahrunfall eine erhebliche Verletzungsgefahr für den Fahrer durch die Armstützen erfolgt.

Die bekannten Vorrichtungen haben darüber hinaus den Nachteil, dass sie die Bewegungsfreiheit der Person erheblich einschränken. Lösungen mit einem Korsett oder dergleichen sind sehr aufwendig und für den alltäglichen Gebrauch wenig geeignet.

Die US 3,063,752 A offenbart eine Stützvorrichtung für den Körper einer Person, welche eine linke und eine rechte mit dem Sitz verbundene höhenveränderliche Achselstütze aufweist. Jede Achselstütze ist dabei auf einem stangenartigen, auf einem Sitzteil befestigten Halter angeordnet, wobei jeder Halter um eine im Wesentlichen zur Vorderkante des Sitzteiles parallele Querachse schwenkbar angeordnet ist. Eine ähnliche Körperstützvorrichtung ist auch aus der US 2,667,913 A, der US 2,667,917 A und der US 4,565,409 A bekannt.

Die US 3,004,794 A zeigt eine orthopädische Unterlage mit Armstützen, welche schwenkbar und höhenverstellbar auf der Unteralge angebracht sind.

Auch diese bekannten Stützeinrichtungen sind für den Einsatz in einem Fahrzeug wenig geeignet, da sie die Bewegungsfreiheit einer in einem Fahrzeug sitzenden Person, beispielsweise einem Kraftfahrzeuglenker, in nicht akzeptabler Weise stark einschränken würden. Eine zu starke Einschränkung der Bewegungsfreiheit kann allerdings die Sicherheit gefährden.

Aufgabe der Erfindung ist es, diese Nachteile zu vermeiden und eine einfach handhabbare Vorrichtung zur Wirbelsäulenentlastung vorzuschlagen, mit der die Bewegungsfreiheit der zu stützenden Person so wenig wie möglich eingeschränkt wird.

Erfindungsgemäß wird dies dadurch erreicht, dass die Stützenhöhen der Achselstützen, vorzugsweise die Längen der Halter, bei einer Wankbewegung des Oberkörpers der Person um eine Längsachse des Sitzes veränderbar sind, wobei die beiden Halter miteinander gekoppelt sind.

Um der Person die Benutzung des Sitzes zu erleichtern, kann vorgesehen sein, dass zumindest ein Halter um eine, vorzugsweise parallel zur Wankachse des Fahrzeugs ausgebildete, Längsachse des Sitzes seitlich schwenkbar ist.

Um eine individuelle Anpassung an die Größe der Person zu ermöglichen und größt mögliche Bewegungsfreiheit zu erreichen, ist es vorteilhaft, wenn der Halter aus mehreren teleskopartig ineinander verschiebbaren Halteteilen besteht. Dabei kann vorgesehen sein, dass die Position zumindest eines ersten und eines zweiten Halteteiles zueinander durch eine Arretiervorrichtung verstellbar ist.

Vorzugsweise ist vorgesehen, dass jeder Halter beispielsweise über eine Schiene, in Richtung einer Hochachse des Sitzes am Sitzteil geführt ist.

Um eine möglichst uneingeschränkte Bewegung der zu stützenden Person zu ermöglichen, ist es besonders vorteilhaft, wenn der linke Halter und der rechte Halter mechanisch, elektrisch, pneumatisch oder hydraulisch miteinander so gekoppelt sind, dass die Summe der Längen des linken Halters und des rechten Halters konstant ist.

Dies kann bei Haltern mit mehreren teleskopartig verfahrbaren Halterteilen dadurch geschehen, dass mit Flüssigkeit oder Gas gefüllte Druckräume innerhalb zumindest zweier Halteteile des linken und des rechten Halters hydraulisch oder pneumatisch über eine Druckleitung miteinander verbunden sind. Alternativ dazu kann gemäß einer einfachen Ausführungsvariante der Erfindung vorgesehen sein, dass der linke und der rechte Halter über einen am Sitz oder am Fahrzeug drehbar gelagerten, vorzugsweise gleicharmigen, Ausgleichshebel verbunden sind.

Ein hoher Sitzkomfort der Person lässt sich erreichen, wenn die Achselstütze um eine Hochachse schwenkbar mit dem Halter verbunden ist. Besonders vorteilhaft ist es dabei, wenn die Armstützen durch Torussegmente gebildet werden. Dadurch wird eine nachteilige Beeinflussung der Blutzirkulation vermieden.

In weiterer Ausführung der Erfindung kann vorgesehen sein, dass die Halter elektrisch ein- und ausfahrbar sind.

Zur weiteren Entlastung der Wirbelsäule und zur Hebung des Komforts kann weiters im Rahmen der Erfindung vorgesehen sein, dass jeder Halter eine vorzugsweise höhenverstellbare Armlehne aufweist. Die Höhenverstellung kann manuell oder elektrisch erfolgen.

Bei Anwendungen für Fahrzeugsitze werden die besten Ergebnisse für die Entlastung der Wirbelsäule erzielt, wenn die Halter und/oder Achselstützen nur wenig elastisch nachgiebig bzw. gefedert ausgeführt sind. Bei Büromöbel oder dergleichen kann es aber durchaus vorteilhaft sein, den Halter oder die Achselstütze mit einem Federelement und/oder Dämpfungselement auszustatten, welches bei plötzlicher Belastung der Achselstütze oder des Halters nachgibt. Besonders vorteilhaft ist es, wenn die Feder- und/oder die Dämpfungscharakteristik an die jeweilige Person angepasst werden kann.

Vorzugsweise ist vorgesehen, dass jeder Halter um eine im Wesentlichen zur Vorderkante des Sitzteiles etwa parallele Querachse schwenkbar angeordnet ist.

Die Erfindung wird im Folgenden anhand der Figuren näher erläutert. Es zeigen:
- Fig. 1: eine an einem Sitz eines Fahrzeugs befestigte erfindungsgemäße Vorrichtung zur Wirbelsäulenentlastung in einer ersten Ausführungsvariante im Gebrauch in einer Seitenansicht;
- Fig. 2: die Vorrichtung im Detail;
- Fig. 3: einen Sitz mit einer erfindungsgemäße Vorrichtung zur Wirbelsäulenentlastung in einer zweiten Ausführungsvariante in einer Seitenansicht; und
- Fig. 4: den Sitz samt Vorrichtung in einer Frontalansicht.

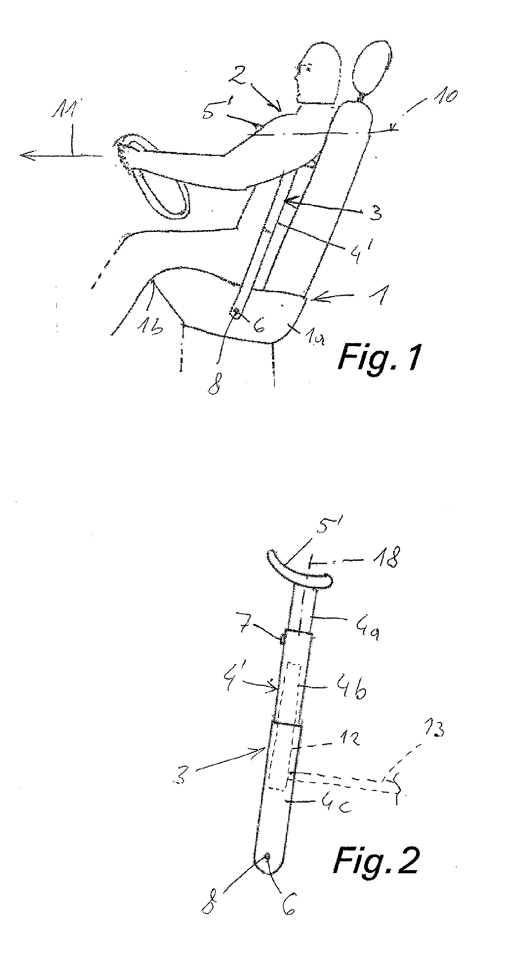

Fig. 1 zeigt eine auf einem Sitz 1 eines nicht weiter dargestellten Fahrzeuges sitzende Person 2, welche durch eine Vorrichtung 3 zur Wirbelsäulenentlastung gestützt ist. Die Vorrichtung 3 besteht aus zwei im Wesentlichen stabförmigen teleskopartig ausfahrbaren Haltern 4', 4", auf welchen torusförmige Achselstützen 5', 5" angeordnet sind. Die Achselstützen 5', 5" sind um eine Hochachse 18 schwenkbar und können zumindest um eine etwa 45° zum Körper der Person 2 gedreht werden, so dass diese am Körper der Person 2 anliegen. Die Halter 4', 4" sind beidseits des Sitzteiles 1a befestigt, wobei die Halter 4', 4" um eine im Wesentlichen parallel zur Sitzkante 1b angeordnete Querachse 6 schwenkbar sind.

Die beiden Armstützen samt Halter 4', 4" nehmen einen Teil des Körpergewichts unter der Achsel der Person 2 auf. Dadurch greifen Stoßbeschleunigungen, wie sie durch Fahrbahnunebenheiten verursacht werden, nicht mehr alleine am Gesäß, sondern auch an den Achseln der Person 2 an. Dadurch wird die Wirbelsäule von der Hauptbelastung durch die schädlichen Stöße verschont. Dadurch, dass die Halter 4', 4" unterhalb des Hüftgelenks der Person 2 beidseits am Sitzteil 1a des Fahrersitzes 1 über ein Gelenk 8, vorzugsweise ein Kugelgelenk, drehbar fixiert sind, kann der Oberkörper der Person 2 nach vorne und auch nach der Seite gebeugt werden. Weiters ermöglicht eine spezielle Bauart der Halter 4', 4" auch die Drehung des Schultergürtels um eine Längsachse 10 etwa parallel zur Fahrtrichtung 11.

Die Achselstütze 5', 5" stützt den Körper der Person 2 im Bereich der Achsel. Er weist im Wesentlichen die Form eines Torussegmentes auf, wodurch ein Abschnüren von Blutgefäßen vermieden wird.

Der Halter 4', 4" besteht aus drei ineinander teleskopartig verschiebbaren Teilen. Die Verbindung zwischen dem Oberteil 4a und dem Mittelteil 4b ist während der Benutzung über eine Arretiereinrichtung 7 fixierbar. Mit dieser Arretiereinrichtung wird die Länge jedes Halters 4', 4" an die Körpergröße der Person 2 angepasst.

Der Mittelteil 4b und der Unterteil 4c des Halters 4', 4" der beiden Armstützen 5 sind mechanisch, pneumatisch, hydraulisch oder elektrisch miteinander gekoppelt, so dass die Summe der Längen der beiden Halter 4', 4" konstant bleibt. Dabei können beispielsweise mit Flüssigkeit oder Gas oder Luft gefüllte Druckräume 12 innerhalb des Mittelteils 4b und des Unterteils 4c des linken Halters 4' und des rechten Halters 4" über eine Druckleitung 13 hydraulisch bzw. pneumatisch miteinander verbunden sein. Wird beispielsweise die linke Armstütze 5' stärker belastet und die rechte Armstütze 5" dafür entlastet, so wird der linke Halter 4' um einen bestimmten Betrag verkürzt und der rechte Halter 4" um den gleichen Betrag verlängert. Die Summe der beiden Stützenhöhen h', h" bleibt somit konstant.

Der Unterteil 4c des Halters 4', 4" ist durch das Gelenk 8 an den Sitzteil des Autositzes fixiert. Das Gelenk 8 ermöglicht eine Schwenkbewegung des Halters 4', 4" vorwärts und rückwärts und in geringem Umfang auch seitwärts.

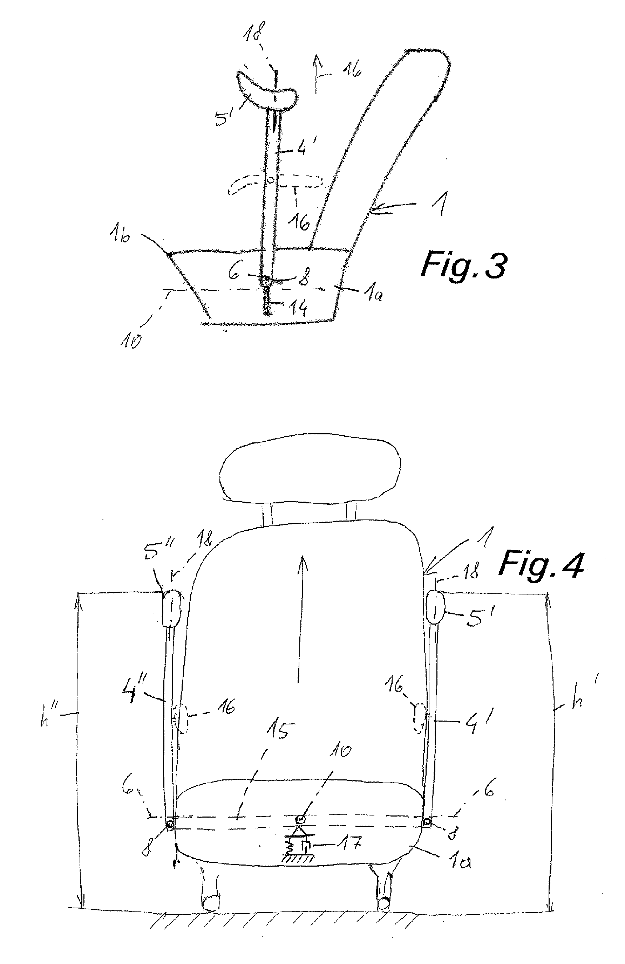

Die Fig. 3 und Fig. 4 zeigen eine einfache Ausführung einer Vorrichtung 3 zur Wirbelsäulenentlastung, wobei die Halter 4', 4" jeweils nur aus einem Halterteil 4a bestehen, welcher über eine Schiene 14 verschiebbar in Richtung der Hochachse 16 des Sitzes 1 mit dem Sitzteil 1a verbunden ist. Um den gewünschten Ausgleich zwischen linker und rechter Achselstütze 5', 5" zu erreichen, sind die Halter 4', 4" innerhalb des Sitzteiles 1a über einen zweiarmigen Ausgleichshebel 15 miteinander verbunden, wobei der Ausgleichshebel 15 mittig schwenkbar um eine Längsachse 11 fest am Sitz 1 oder am Fahrzeug gelagert ist. Durch den Ausgleichshebel 15 ist ebenfalls eine gegenläufige Höhenverstellung der linken und der rechten Achselstütze 5', 5" möglich, wobei die Summe der Stützenhöhen h', h" konstant bleibt.

Durch die beschriebene Vorrichtung 3 zur Wirbelsäulenentlastung kann der Rumpf der Person 2 nach vorne gebeugt werden. Wird die Vorrichtung 3 in einem Fahrzeug eingesetzt, so sind alle Armaturen und Bedienelemente des Fahrzeugs problemlos erreichbar. Der Rumpf der Person 2 kann auch nach der Seite gebeugt werden. Durch die dynamische Veränderbarkeit der Stützenhöhen kann auch der Schultergürtel um eine Längsachse 10 parallel zur Fahrtrichtung 11 gedreht werden. Zur Hebung des Komforts kann die Armstütze 5', 5" aus weichem, anpassungsfähigem Material bestehen. Eventuell kann eine ein Gel enthaltende Polsterung verwendet werden. Insbesondere bei stationären Sitzgelegenheiten kann zumindest ein Feder- und/oder Dämpfungselement 17 im Bereich der Lagerung 18 oder im Bereich der Halter 4', 4" oder Armstützen 5', 5" von Vorteil sein, um Stossbelastungen beispielsweise beim Hinsetzen der Person abzumildern. Weiters können zur weiteren Entlastung der Wirbelsäule und zur Erhöhung des Komforts an den Haltern 4', 4" höhenverstellbare Armlehnen 16 vorgesehen sein.

Durch die Schwenkbarkeit und Beweglichkeit der Halter 4', 4" ist ein behinderungsfreies Ein- und Aussteigen der Person aus dem Fahrzeug möglich.

Die erfindungsgemäße Vorrichtung 3 eignet sich nicht nur für Fahrzeugsitze, sondern auch für andere Sitzgelegenheiten wie Bürosessel, Rollstühle oder dergleichen.

## Patentansprüche

1. Vorrichtung (3) zur Wirbelsäulenentlastung einer auf einem Sitz (1), insbesondere einem Fahrzeugsitz, sitzenden Person (2), wobei die Vorrichtung (3) eine linke und eine rechte mit dem Sitz (1) verbundene höhenveränderliche Achselstütze (5', 5") aufweist, wobei jede Achselstütze (5', 5") auf einem vorzugsweise stangenartigen Halter (4', 4") angeordnet ist, wobei vorzugsweise der Halter (4', 4") am Sitzteil (1a) des Sitzes (1) befestigt ist, **dadurch gekennzeichnet, dass** die Stützenhöhen (h', h") der Achselstützen (5', 5"), vorzugsweise die Längen der Halter (4', 4"), bei einer Wankbewegung des Oberkörpers der Person (2) um eine Längsachse (10) des Sitzes (1) veränderbar sind, wobei die beiden Halter (4', 4") miteinander gekoppelt sind.

2. Vorrichtung (3) nach Anspruch 1, **dadurch gekennzeichnet, dass** zumindest ein Halter (4', 4") um eine, vorzugsweise parallel zur Fahrtrichtung (11) des Fahrzeugs ausgebildete, Längsachse (10) des Sitzes (1) seitlich schwenkbar ist.

3. Vorrichtung (3) nach Anspruch 1 oder 2, **dadurch gekennzeichnet, dass** jeder Halter (4', 4") aus zumindest zwei, vorzugsweise aus drei, teleskopartig ineinander verschiebbaren Halteteilen (4a, 4b, 4c) besteht.

4. Vorrichtung (3) nach Anspruch 3, **dadurch gekennzeichnet, dass** die Position zumindest eines ersten und eines zweiten Halteteiles (4a, 4b) zueinander durch eine Arretiervorrichtung (7) verstellbar ist.

5. Vorrichtung (3) nach einem der Ansprüche 1 bis 4, **dadurch gekennzeichnet, dass** jeder Halter (4', 4") vorzugsweise über eine Schiene (14), in Richtung einer Hochachse (16) des Sitzes (1) am Sitzteil (1a) geführt ist.

6. Vorrichtung (3) nach einem der Ansprüche 1 bis 5, **dadurch gekennzeichnet, dass** der linke Halter (4') und der rechte Halter (4") mechanisch, elektrisch, pneumatisch oder hydraulisch miteinander so gekoppelt sind, dass die Summe der Stützenhöhen (h', h") des linken Halters (4') und des rechten Halters (4") konstant ist.

7. Vorrichtung (3) nach Anspruch 6, **dadurch gekennzeichnet, dass** mit Flüssigkeit oder Gas gefüllte Druckräume (12) innerhalb zumindest zweier Halteteile (4a, 4b) des linken und des rechten Halters (4', 4") hydraulisch oder pneumatisch über eine Druckleitung (13) miteinander verbunden sind.

8. Vorrichtung (3) nach Anspruch 6, **dadurch gekennzeichnet, dass** der linke Halter (4') und der rechte Halter (4") über einen am Sitz (1) oder am Fahrzeug drehbar gelagerten, vorzugsweise gleicharmigen, Ausgleichshebel (15) verbunden sind.

9. Vorrichtung (3) nach einem der Ansprüche 1 bis 8, **dadurch gekennzeichnet, dass** die Achselstütze (5', 5") um eine Hochachse (18) schwenkbar mit dem Halter (4) verbunden ist.

10. Vorrichtung (3) nach einem der Ansprüche 1 bis 9, **dadurch gekennzeichnet, dass** die Achselstützen (5', 5") durch Torussegmente gebildet sind.

11. Vorrichtung (3) nach einem der Ansprüche 1 bis 10, **dadurch gekennzeichnet, dass** die Halter (4', 4") elektrisch ein- und ausfahrbar sind.

12. Vorrichtung (3) nach einem der Ansprüche 1 bis 11, **dadurch gekennzeichnet, dass** jeder Halter (4', 4") eine vorzugsweise höhenverstellbare Armlehne (16) aufweist.

13. Vorrichtung (3) nach einem der Ansprüche 1 bis 12, **dadurch gekennzeichnet, dass** zumindest ein Feder- und/oder Dämpfungselement (17) vorgesehen ist, um vertikale Stossbelastungen abzufedern, wobei vorzugsweise die Feder- und/oder Dämpfungseigenschaften verstellbar sind.

14. Vorrichtung (3) nach einem der Ansprüche 1 bis 13, **dadurch gekennzeichnet, dass** jeder Halter (4', 4") um eine im Wesentlichen zur Vorderkante (1b) des Sitzteiles (1a) etwa parallele Querachse (6) schwenkbar angeordnet ist.
